Europäisches Patentamt

European Patent Office

Office européen des brevets

(11) Veröffentlichungsnummer: **0 095 075**
A1

# (12) EUROPÄISCHE PATENTANMELDUNG

(21) Anmeldenummer: 83104435.9

(22) Anmeldetag: 05.05.83

(51) Int. Cl.³: **A 61 B 10/00**

(30) Priorität: 21.05.82 DE 3219271

(43) Veröffentlichungstag der Anmeldung:
30.11.83 Patentblatt 83/48

(84) Benannte Vertragsstaaten:
AT BE CH DE FR GB IT LI NL SE

(71) Anmelder: SIEMENS AKTIENGESELLSCHAFT
Berlin und München Wittelsbacherplatz 2
D-8000 München 2(DE)

(72) Erfinder: Hetz, Walter
Adam-Kraft-Strasse 17
D-8520 Erlangen(DE)

(72) Erfinder: Weber, Peter
Am Eichengarten 3
D-8520 Buckenhof(DE)

(54) Ultraschall-Applikator.

(57) Ein Ultraschall-Applikator, der sich insbesondere zur Tastuntersuchung von Körperhöhlen od.dgl. eignet, besteht aus einem Ultraschallkopf (1,11,21,31), der von einer Untersuchungsperson handhabbar ist, und einem Kabel (10,20,30,40) zum Anschluss an ein Bildsignalverarbeitungsgerät.

Der Ultraschallkopf (1, 11, 21, 31) ist zur Anpassung an ein Führungsorgan, insbesondere an der Finger der Untersuchungsperson, ausgebildet.

FIG 4

EP 0 095 075 A1

Croydon Printing Company Ltd

SIEMENS AKTIENGESELLSCHAFT          Unser Zeichen
Berlin und München                  VPA 82 P 3755 E

Ultraschall-Applikator

Die Erfindung bezieht sich auf einen Ultraschall-Applikator für die Ultraschall-Abtastung, insbesondere von Körperhöhlen od.dgl., mit einem Ultraschallkopf, der von einer Untersuchungsperson handhabbar ist, und einer Anschlußleitung zum Anschluß an ein Bildsignalverarbeitungsgerät.

Es sind bereits Ultraschall-Applikatoren dieser Art bekannt, die jedoch alle nur zur Abtastung von körperinternen Organen von der unversehrten Körperoberfläche her ausgebildet sind. Weiterhin wurde vorgeschlagen, auch solche Ultraschall-Applikatoren aufzubauen, die intra-operativ eingesetzt werden können. Auch für die Urologie und Gynäkologie besteht das Bedürfnis, Ultraschall-Applikatoren für die Untersuchung von Körperhöhlen auszubilden, so daß sie insbesondere als Hilfsmittel für solche Untersuchungen verwendet werden können, bei denen bisher die Diagnose aufgrund eines manuellen Tastbefundes gestellt wurde.

Aufgabe der Erfindung ist es daher einen solchen Ultraschall-Applikator zu schaffen, der als Hilfsmittel für die Untersuchung von Körperhöhlen geeignet ist.

Die Aufgabe wird erfindungsgemäß dadurch gelöst, daß der Ultraschallkopf zur Anpassung an ein Führungsorgan, insbesondere an den Finger der Untersuchungsperson, ausgebildet ist.

Wht 5 Rl / 18.05.1982

Ein Ultraschall-Applikator nach der Erfindung kann also die routinemäßig durchgeführte Tastuntersuchung um eine Ultraschall-Abbildung erweitern. Es können somit Befunde erhoben werden, die sich bisher entweder durch ihre kleine räumliche Ausdehnung oder durch ihre tiefe Lage dem manuellen Tastbefund entziehen.

Ein gemäß der Erfindung ausgebildeter Ultraschallkopf läßt sich in einfachster Weise zusammen mit dem Führungsorgan in eine beliebige Applikationslage bringen. Bezüglich eines zu untersuchenden Körperkanals bedeutet dies, daß der Ultraschall-Applikator beliebig rück-, vorder- oder seitenwandig an die abzutastende Kanalwandlung angelegt werden kann. Der erfindungsgemäße Ultraschall-Applikator eignet sich neben dem urologischen und gynäkologischen Einsatz auch für die Untersuchung anderer Körperhöhlen.

Weitere Vorteile und Einzelheiten der Erfindung ergeben sich aus der nachfolgenden Beschreibung von Ausführungsbeispielen anhand der Zeichnung in Verbindung mit den Unteransprüchen.

Es zeigen:

Fig. 1 und 2 zwei verschiedene Ansichten eines ersten Ultraschall-Applikators,

Fig. 3 einen zweiten Ultraschall-Applikator,

Fig. 4 eine bevorzugte Applikationsmöglichkeit der Ultraschall-Applikatoren gemäß Fig. 1 bis 3 bei der Abtastung eines Körperkanals,

Fig. 5 einen mit einem Handgriff komplettierten Ultraschall-Applikator.

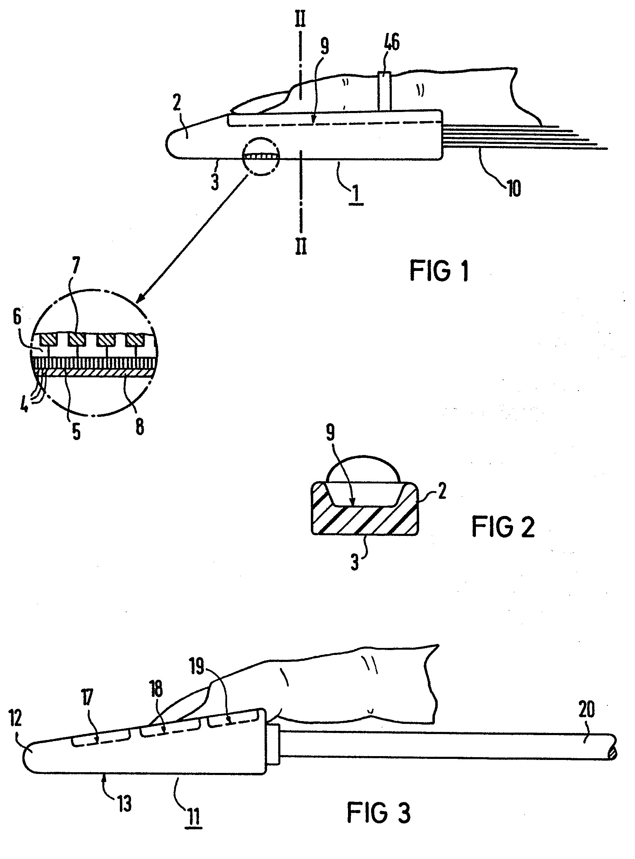

In der Figur 1 kennzeichnet 1 einen Ultraschall-Applikator mit Ultraschall-Array, 2  das Applikatorgehäuse mit einer Applikationsfläche 3. Das  Applikatorgehäuse 2 umfaßt dabei an der Applikationsfläche 3, wie in der Ausschnittsvergrößerung dargestellt ist, eine Vielzahl von Wandlerelementen 4, die im vorliegenden Ausführungsbeispiel feingeteilt sind. Immer mehrere dieser feingeteilten Wandlerelemente 4, z.B. im vorliegenden Fall insgesamt vier Wandlerlelemente, sind elektrisch zu je einer Gruppe 5 (von z.B. insgesamt 48 Gruppen) zusammenkontaktiert. Zur Gruppenkontaktierung dienen Kontaktfahnen 6 eines Kontaktkammes. Mit 7 ist ein Trägerkörper für die Ultraschall-Wandlerelemente 4 bezeichnet. Der Trägerkörper, in den die Kontaktfahnen 6 eingebettet sind, besteht beispielsweise aus Epoxydharz mit eingebrachtem oxidiertem Wolframpulver. Mit 8 ist eine Anpassungsschicht für die Wandlerelemente bezeichnet. Die Anpassungsschicht kann aus Epoxydharz bestehen. Das Grundprinzip der Feinteilung von Wandlerelementen ist z.B. in der US-PS 43 05 014 näher beschrieben.

Am rückwärtigen Teil des Applikatorgehäuses 1 ist ein Signalanschlußkabel 10 geführt. Das Signalanschlußkabel 10 ist derart gelagert, daß es dem Ultraschall-Applikator 1 praktisch zugfrei in jede beliebige Abtastlage folgen kann. Beispielsweise hat das Kabel 10 im Anschlußbereich einen etwa sichelförmigen Querschnitt, wobei die Einzelleitungen nebeneinander liegen und vom Umfang des Fingers der Untersuchungsperson großflächig an der Handfläche geführt werden können.

Aus den Figuren 1 und 2 ist ersichtlich, daß das Applikatorgehäuse 1 an der dem Ultraschall-Array gegenüberliegenden Seite eine Ausnehmung 9 aufweist. Insbesondere die Schnittdarstellung der Figur 2 zeigt, daß dafür
die rückseitige    Fläche des Applikatorgehäuses 2
konkav eingewölbt ist und eine Fingermulde bildet. In
die Auswölbung 9 kann also der Finger der Untersuchungsperson eingefügt werden, wobei bei weitgehender Anpassung von Finger und Applikatorgehäuse Tastuntersuchungen mit am Finger applizierten Ultraschall-
Applikator möglich sind. Zur Fixierung des Ultraschall-
Applikators 1 am Finger der Untersuchungsperson kann
zusätzlich ein Bügel 46 vorgesehen sein, der im einfachsten Fall durch einen elastischen Gummizug gebildet
wird. Der gleiche Zweck wird auch durch Fingerling aus
ultraschall-durchlässigem Material, beispielsweise
Latex, erfüllt, der über den am Finger angebrachten
Applikator 1 gezogen wird.

In der Figur 3 kennzeichnet 11 einen Ultraschall-
Applikator mit einem Applikatorgehäuse 12 und der
Applikationsfläche 13. An der rückwärtigen Fläche
sind nun jeweils drei Fingermulden 17, 18, 19 gebildet. Die Fingermulden 17 bis 19 sind jeweils
durch Stege getrennt, so daß in verschiedenem Abstand einen Halt für den Führungsfinger der Untersuchungsperson gegeben ist. Mit 20 ist ein Anschlußkabel bezeichnet, das in diesem Fall als Rundkabel
mit üblicher Zugentlastung an dem Applikatorgehäuse
11 angebracht ist.

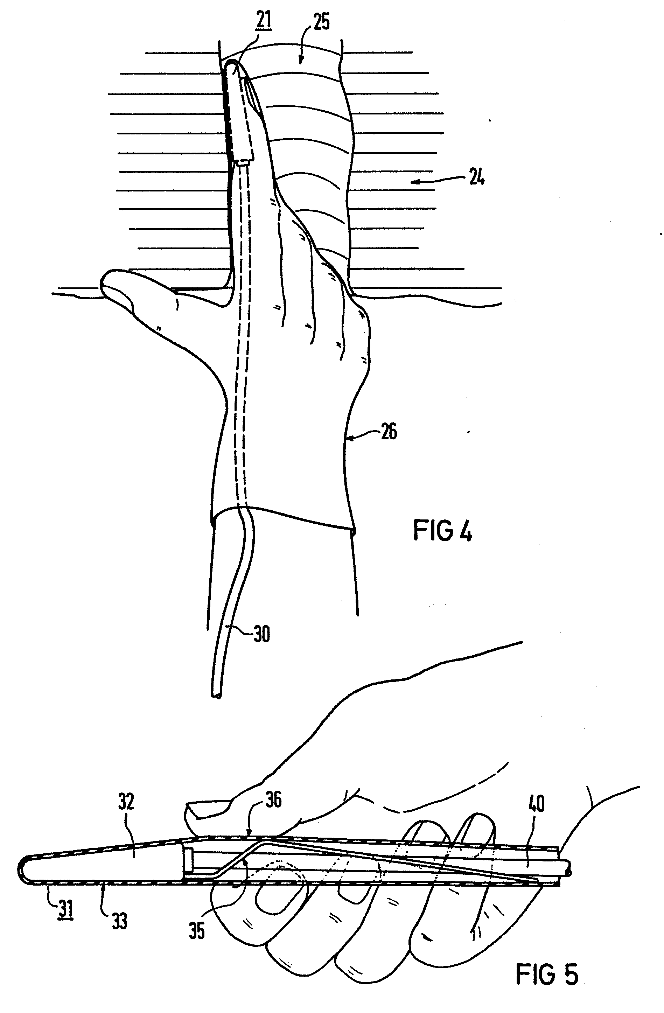

Die Ultraschall-Applikatoren der Figuren 1 bis 3
eignen sich insbesondere für einen urologischen und
gynäkologischen Einsatz, bei dem üblicherweise manuelle
Tastbefunde durchgeführt werden. Dabei wird mit dem
Zeigefinger die Kanalwandung, beispielsweise des Rektums,
der Urethra  oder der Vagina, abgetastet. Dies ist beispielhaft in der Figur 4 dargestellt: Mit 24 ist eine Körperregion mit einem Körperkanal 25 bezeichnet. Die Hand der Untersuchungsperson trägt einen ultraschalldurchlässigen Handschuh 26 aus Latex od.dgl., in den ein Ultraschall-Applikator 21 mit Anschlußkabel 30 eingebracht ist. Es wird also gewissermaßen eine Tastuntersuchung durchgeführt, welche durch das nun erzeugbare Ultraschall-Bild des zu untersuchenden Bereiches zu einer wesentlich genaueren Diagnose führen kann.

Gegebenenfalls können auch tiefere Körperkanäle mit dem erfindungsgemäßen Applikator untersucht werden. Dafür ist ein zusätzlicher Handgriff als Manipulationshilfe verwendbar. Es wird ebenfalls eine Abtastung im weiteren Sinne durchgeführt: In der Figur 5 ist an einem Gehäuse 32 eines Ultraschall-Applikators 31 ein Metallbügel 35 angebracht. Um den gesamten Ultraschall-Applikator 31 und Metallbügel 35 einschließlich Kabelanschluß 40 ist eine ultraschalldurchlässige Ummantelung 36, beispielsweise aus Latex, angebracht. Es wird dadurch ein Handgriff für den Ultraschall-Applikator 31 gebildet, wobei durch die Umhüllung die Sterilität des gesamten Instrumentes gewährleistet wird. Der Metallbügel 35 kann eine vorgegebene Geometrie aufweisen, beispielsweise abgekröpft sein, so daß bestimmte Halterungspositionen vorgegeben sind. Die Enden des Metallbügels 35 sind im Applikatorgehäuse 32 einsteckbar oder anklemmbar, so daß der Handgriff leicht ausgetauscht werden kann.

Es ist also mit einem derartigen Instrument insbesondere eine Abtastung von bisher nicht erreichbaren Körperregionen möglich. Es können nun auch solche Tastbefunde erzielt werden, die sich bisher durch ihre tiefe Lage dem normalen Fingertastbefund entzogen haben.

5 Figuren, 9 Patentansprüche

<u>Patentansprüche</u>

1. Ultraschall-Applikator für die Ultraschall-Abtastung, insbesondere von Körperhöhlen od.dgl., mit einem Ultraschallkopf, der von einer Untersuchungsperson handhabbar ist, und einer Anschlußleitung zum Anschluß an ein Bildsignalverarbeitungsgerät, d a d u r c h g e - k e n n z e i c h n e t , daß der Ultraschallkopf (1, 11, 21, 31) zur Anpassung an ein Führungsorgan, insbesondere an den Finger der Untersuchungsperson, ausgebildet ist.

2. Ultraschall-Applikator nach Anspruch 1, d a - d u r c h g e k e n n z e i c h n e t , daß der Ultraschallkopf (1,11,21) mit der rückwärtigen Fläche des Schallkopfgehäuses (2,12) an das Führungsorgan angepaßt ist.

3. Ultraschall-Applikator nach Anspruch 1, d a - d u r c h g e k e n n z e i c h n e t , daß die rückwärtige Fläche des Schallkopfgehäuses (2, 12) wenigstens teilweise konkav gewölbt ist.

4. Ultraschall-Applikator nach Anspruch 3, d a - d u r c h g e k e n n z e i c h n e t , daß einzelne Fingermulden (17, 18, 19) in der rückwärtigen Fläche des Schallkopfgehäuses (12) durch Stege getrennt sind.

5. Ultraschall-Applikator nach Anspruch 1, d a - d u r c h g e k e n n z e i c h n e t , daß an das Schallkopfgehäuse (32) Führungsgriffe (35) vorgegebener Geometrie anklemmbar oder ansteckbar sind.

6. Ultraschall-Applikator nach einem der Ansprüche 1 bis 4, d a d u r c h   g e k e n n z e i c h n e t , daß das Schallkopfgehäuse (2) einschließlich der Anschlußkabel (12) in einen Handschuh (26) für die Untersuchungsperson aus ultraschalldurchlässigem Material, beispielsweise Latex, einbringbar ist.

7. Ultraschall-Applikator nach Anspruch 5, d a - d u r c h   g e k e n n z e i c h n e t , daß Schallkopfgehäuse (32) und Führungsgriff (35) mit einer ultraschalldurchlässigen Umhüllung, beispielsweise aus Latex, versehen ist.

8. Ultraschall-Applikator nach einem der Ansprüche 1 bis 4, d a d u r c h   g e k e n n z e i c h n e t , daß die Anschlußleitung (12) durch ein Flachkabelteil (10) gebildet wird.

9. Ultraschall-Applikator nach einem der Ansprüche 1 bis 4, d a d u r c h   g e k e n n z e i c h n e t , daß am Schallkopfgehäuse ein Bügel (46) zur Fixierung des Ultraschallkopfes (1) am Finger der Untersuchungsperson vorhanden ist.

0095075

FIG 1

FIG 2

FIG 3

FIG 4

FIG 5

Europäisches
Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-4 327 738 (P.S. GREEN et al.) <br> * Zusammenfassung; Spalte 3, Zeilen 10-26; Spalte 4, Zeilen 18-31, 51-59; Spalte 7, Zeile 57 - Spalte 8, Zeile 15; Abbildungen 1-3 * | 1 | A 61 B 10/00B |
| | --- | | |
| Y | US-A-4 250 894 (E.H. FREI et al.) <br> * Zusammenfassung; Spalte 4, Zeilen 8-16, 45-52; Spalte 5, Zeilen 29-57; Spalte 5, Zeile 64 - Spalte 6, Zeile 2; Spalte 9, Zeilen 25-37; Abbildungen 1-6, 12 * | 1,6 | |
| | --- | | |
| A | US-A-3 827 115 (N. BOM) <br> * Zusammenfassung; Spalte 2, Zeilen 48-55; Spalte 5, Zeilen 63-68; Spalte 7, Zeilen 9-26, 38-43, 64-66; Abbildungen 1,8 * | 1,7 | **RECHERCHIERTE SACHGEBIETE (Int. Cl. ³)** <br><br> A 61 B 10/00 <br> A 61 B 1/30 <br> A 61 B 5/04 |
| | --- | | |
| A | US-A-4 244 375 (A.O. FARRAR et al.) <br> * Zusammenfassung; Spalte 5, Zeile 67 - Spalte 6, Zeile 5; Spalte 7, Zeilen 20-38; Spalte 7, Zeile 63 - Spalte 8, Zeile 20; Abbildungen 2A,3A,4,5 * | 2,3,5, 9 | |
| | ---     -/- | | |

Der vorliegende Recherchenbericht wurde für alle Patentansprüche erstellt.

| Recherchenort <br> DEN HAAG | Abschlußdatum der Recherche <br> 23-08-1983 | Prüfer <br> RIEB D.K. |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-A-2 513 445 (J.P. GOUS)<br>* Seite 5, Zeilen 12-20; Abbildung 1 *<br><br>----- | 3,4 | |

**RECHERCHIERTE SACHGEBIETE (Int. Cl. ³)**

Der vorliegende Recherchenbericht wurde für alle Patentansprüche erstellt.

| Recherchenort<br>DEN HAAG | Abschlußdatum der Recherche<br>23-08-1983 | Prüfer<br>RIEB D.K. |
|---|---|---|